# EUROPÄISCHE PATENTSCHRIFT

(11) **EP 0 691 335 B1**
(45) Veröffentlichungstag und Bekanntmachung des Hinweises auf die Patenterteilung: **03.05.2000**
(21) Anmeldenummer: 95109933.2
(22) Anmeldetag: 26.06.1995
(51) Int. Cl.: C07D 307/60

(54) **Verfahren zur Herstellung von Bernsteinsäureanhydrid**
Process for the preparation of succinic anhydride
Procédé pour la préparation de l'anhydride succinique

(30) Priorität: 08.07.1994 DE 4424069
(43) Veröffentlichungstag der Anmeldung: 10.01.1996
(73) Patentinhaber: BAYER AG, 51368 Leverkusen (DE)
(72) Erfinder: Jentsch, Joerg-Dietrich, Dr., D-45468 Mülheim a.d.R. (DE); Martin, Georg, Dr., D-40219 Düsseldorf (DE); Zirngiebl, Eberhard, Dr., D-51061 Köln (DE)

(56) Entgegenhaltungen:
- DE-B- 1 768 791
- DE-C- 441 002
- GB-A- 920 506
- GB-A- 1 050 031
- US-A- 2 245 404
- CHEMICAL ABSTRACTS, vol. 103, no. 1, 8. Juli 1985, Columbus, Ohio, US; abstract no. 6210k, & CS-A-218 083 (J. SRAGA ET AL.)
- CHEMICAL ABSTRACTS, vol. 79, no. 1, 9. Juli 1973, Columbus, Ohio, US; abstract no. 5013x, & JP-B-48 007 609 (MITSUBISHI PETROCHEMICAL CO., LTD.)
- CHEMICAL ABSTRACTS, vol. 118, no. 23, 7. Juni 1993, Columbus, Ohio, US; abstract no. 233869k, & CN-A-1 063 484 (X. TANG)
- SOVIET PATENTS ABSTRACTS Week 9034, 3. Oktober 1990 Derwent Publications Ltd., London, GB; AN 90-259197 & SU-A-1 541 210 (AS USSR BASHKIRSK)
- CHEMICAL ABSTRACTS, vol. 93, no. 7, 18. August 1980, Columbus, Ohio, US; abstract no. 71047b, & SU-A-721 406 (INSTITUTE OF ORGANIC SYNTHESIS, ACADEMY OF SCIENCES, LATVIAN)
- CHEMICAL ABSTRACTS, vol. 98, no. 5, 31. Januar 1983, Columbus, Ohio, US; abstract no. 34252x, & CS-A-195 860 (M. POLIEVKA ET AL.)
- CHEMICAL ABSTRACTS, vol. 114, no. 5, 4. Februar 1991, Columbus, Ohio, US; abstract no. 42561m, & JP-A-02 200 680 (MITSUBISHI KASEI CORP.)
- JOURNAL OF ORGANIC CHEMISTRY, Bd.28, 1963, EASTON US Seiten 498 - 501 J.H. BREWSTER AND AL.

## Beschreibung

Die vorliegende Erfindung betrifft ein Verfahren zur Herstellung von Bernsteinsäureanhydrid (BSA) durch katalytische Hydrierung von Maleinsäureanhydrid (MSA) in flüssiger Phase.

BSA ist ein wertvoller Synthesebaustein zur Herstellung von Bernsteinsäure, Bernsteinsäurediestern, Succinimiden, Polyestern und Pharmaka.

Es ist bereits bekannt, BSA durch Dehydratisierung von Bernsteinsäure oder durch katalytische Hydrierung von Maleinsäureanhydrid (MSA) zu erhalten. Die katalytische Hydrierung von MSA wird im allgemeinen in der flüssigen Phase an einem suspendierten Katalysator vorgenommen.

Hierbei werden milde Reaktionsbedingungen, wie die Mitverwendung eines Verdünnungsmittels, die Mitverwendung von Desaktivierungsmitteln für den Katalysator und die Einstellung niedriger Temperaturen, vorgesehen. Diese milden Reaktionsbedingungen dienen ganz offensichtlich der Zurückdrängung unerwünschter Reaktionen, wie der Polymerisation des MSA oder der Überhydrierung, beispielsweise zum γ-Butyrolacton. So wird gemäß CS 218 083 (zitiert nach C.A. 103, 6210 k) in Essigsäureethylester in Gegenwart von Raney-Nickel, das vorher durch Essigsäure oder durch Essigsäureethylester desaktiviert wurde, gearbeitet.

Gemäß CS 195 860 wird beispielsweise ein 1:1-Gemisch von MSA in γ-Butyrolacton an Katalysatoren aus der Gruppe Kupfer auf Kieselgur, NiO auf Kieselgur, Cu-Zn-Cr-Katalysator oder Raney-Kupfer bei 100 bis 300°C zu einem Reaktionsprodukt umgesetzt, das Bernsteinsäure, γ-Butyrolacton und Propionsäure enthält. Hierbei bildet sich aus dem C₄-Ausgangsmaterial MSA, offenbar durch Decarbonylierung oder Decarboxylierung, das C₃-Produkt Propanol.

Gemäß JP 02/200 680 (zitiert nach C.A. 114, 42561 m) wird bei 200°C in Tetraethylenglykol-dimethylether Maleinsäure oder MSA zu γ-Butyrolacton und BSA umgesetzt; hierbei wird ein kompliziertes Katalysatorsystem aus Ruthenium, organischen Phosphinen, konjugierten Basen von Säuren mit einem pKa < 2 und Chlor enthaltenden Verbindungen aus der Gruppe von Hydrochloriden oder Metallchloriden eingesetzt.

In Aceton als Lösungsmittel wird gemäß SU 1 541 210 (zitiert nach C.A. 113, 23679 u) bei 15 bis 50°C gearbeitet, wobei ein Pd/A-Kohle-Katalysator, der mit Triethylenamin modifiziert worden war, eingesetzt wird. Dioxan als Lösungsmittel wird in SU 721 406 (zitiert nach C.A. 93, 71047 b) vorgeschlagen, worin das MSA als 30 bis 5 gew.-%ige Lösung enthalten ist.

Das Verfahren von JP 48/07609 (1973) arbeitet in der Gasphase und unter Anwendung einer weiteren speziellen Maßnahme, nämlich einem Unterschuß an Wasserstoff (Molverhältnis H₂: MSA = 1:3), wobei gemäß Beispiel 1 dieser JP-Patentanmeldung als Hydrierkatalysator sowohl Pd auf Al₂O₃ als auch ein Ni-Re-Katalysator gemeinsam zur Anwendung kommen.

Gemäß Verfahren von CN 1 063 484 wird zur Herstellung von BSA so verfahren, daß MSA bei 60 bis 80°C aufgeschmolzen wird und die Hydrierung in Gegenwart eines aus vier Komponenten (ohne nähere Bezeichnung) bestehenden Raney-Ni-Katalysators bei steigender Temperatur von 70 bis 126°C durchgeführt wird.

Das Reaktionsprodukt wird anschließend abgekühlt und nach Verfestigung grob zerkleinert und verpackt. Dieses Verfahren vermeidet zwar die Mitverwendung eines Lösungsmittels, beinhaltet dafür aber einen komplizierten (nicht näher beschriebenen) Katalysator und die Notwendigkeit einer komplizierten Temperaturführung, die offensichtlich der zunehmenden Bildung von BSA im Reaktionsgemisch folgt und deren Nichteinhaltung zum Auskristallisieren von BSA und zur Behinderung des Rührers führt.

US-A-2,245,404 offenbart ebenfalls ein Verfahren zur Herstellung von BSA, das bei 55°C arbeitet. Ebenso wird dort offenbart, daß bereits Temperaturen von 125-135°C zu einem Verlust an Katalysatoraktivität führen.

Es wurde nun gefunden, daß entgegen den Vorurteilen bezüglich unerwünschter Nebenreaktionen bei Temperaturen hydriert werden kann, die von Anfang an oberhalb des Schmelzpunkts von BSA liegen, wobei offensichtlich die gewünschte Hydrierreaktion vom MSA zum BSA alle anderen Konkurrenzreaktionen überholt und dennoch unerwünschte weitere Hydrierungen oder Spaltreaktionen nicht eintreten. So werden beispielsweise nur unbedeutendende Mengen an Buttersäure und γ-Butyrolacton beobachtet.

Die Erfindung betrifft demnach ein Verfahren zur Herstellung von Bernsteinsäureanhydrid (BSA) durch katalytische Hydrierung von Maleinsäureanhydrid (MSA) mit Wasserstoff in flüssiger Phase, das dadurch gekennzeichnet ist, daß ein Einsatzmaterial der Zusammensetzung 5 bis 100 Gew.-% MSA und 95 bis 0 Gew.-% BSA, bezogen auf das Gesamtgewicht von MSA und BSA, bei 120 bis 150°C und einem H₂-Druck von 10 bis 150 bar an einem Hydrier-Katalysator umgesetzt wird.

Das erfindungsgemäße Verfahren wird bei einer Temperatur von 120 bis 150°C, bevorzugt bei 125 bis 140°C, besonders bevorzugt bei 127 bis 135°C durchgeführt. Wasserstoff wird im Überschuß angeboten. Seine Menge wird nicht durch das Molverhältnis, sondern, wie bei Hydrierreaktionen unter erhöhtem Druck üblich, durch den H₂-Druck dargestellt, der 10 bis 150 bar, bevorzugt 20 bis 130 bar, besonders bevorzugt 50 bis 120 bar beträgt.

Als Katalysator wird ein solcher eingesetzt, der sich für die Hydrierung von Doppelbindungen als besonders geeignet herausgestellt hat. Ein solcher Katalysator gehört der Gruppe der Raney-Skelettkatalysatoren und der Katalysatoren der I. und VIII. Nebengruppe an. Im einzelnen seien genannt: Raney-(Ni,Co,Cu,Fe), wobei eines oder mehrere der hydrieraktiven Metalle gegenwärtig sein kann, sowie ein Katalysator mit einer oder mehreren hydrieraktiven Komponenten aus der I. und VIII. Nebengruppe, wie Pd, Rh, Ru, Pt, Ir, Ni, Co, Fe und Cu. Während Raney-Katalysatoren der genannten Art als Skelettkatalysatoren eingesetzt werden und somit keinen Träger benötigen, können die Katalysatoren der VIII. und I. Nebengruppe sowohl als Metallpulver (in Form eines Katalysatorschlammes) als auch als Trägerkatalysatoren eingesetzt werden. Träger hierfür sind beispielsweise Aluminiumoxide der verschiedenen Strukturen, SiO₂ der verschiedenen Strukturen, Aktivkohle, Gemische aus Aluminiumoxiden und SiO₂, Titandioxid, Bariumsulfat und andere dem Fachmann bekannte inerte Träger in Form von Pulvern oder Formkörpern.

Unter den Raney-Katalysatoren sind aus Kostengründen diejenigen bevorzugt, bei denen nur eins oder zwei der genannten Metalle, besonders bevorzugt nur eins der genannten Metalle gegenwärtig sind (ist). In ganz besonders bevorzugter Weise wird Raney-Ni eingesetzt.

Unter den Platingruppenmetallen sind Pd oder Pt bevorzugt; besonders bevorzugt ist Pd.

Während das erfindungsgemäße Verfahren grundsätzlich auch in Gegenwart von Verdünnungsmitteln, beispielsweise der oben genannten Art, durchgeführt werden kann, ist es vor allem wegen der Vereinfachung sinnvoll und bevorzugt, es völlig lösungsmittelfrei durchzuführen.

Im erfindungsgemäßen Verfahren wird ein Einsatzmaterial der Zusammensetzung 5 bis 100 Gew.-% MSA und 95 bis 0 Gew.-% BSA, bevorzugt 20-100 Gew.-% MSA und 80-0 Gew.-% BSA, umgesetzt. Es ist somit möglich, reines MSA erfindungsgemäß umzusetzen. Dies kann beispielsweise als Batch-Verfahren, durch Anwendung einer Pfropfströmung in einem Rohrreaktor kontinuierlich oder in Form der ebenfalls kontinuierlichen Rieselphase an einem Festbettkatalysator durchgeführt werden. Das Einsatzmaterial kann jedoch im Rahmen der angegebenen Mischung auch BSA enthalten. Auch diese Verfahrensvariante kann als Batch-Verfahren, unter Durchführung einer Pfropfströmung oder in der Rieselphase an einem Festbettkatalysator durchgeführt werden.

Als spezielle Verfahrensvariante sei die Semibatch-Fahrweise genannt, bei welcher so vorgegangen wird, daß zu einer Suspension des Katalysators in vorgelegtem flüssigem BSA verflüssigtes MSA unter Hydrierbedingungen zugesetzt und hydriert wird, daß darauf BSA in einer Menge abgezogen wird, die dem zugesetzten MSA im Durchschnitt entspricht (+/- 30 %), so daß noch BSA im Reaktor zurückbleibt und daß der Vorgang unter erneutem Zusatz von MSA wiederholt wird. Die Menge des zugesetzten MSA, bezogen auf die Masse an vorgelegtem BSA, kann in weiten Grenzen schwanken, beispielsweise von 5 bis 5 000 %, bevorzugt 100 bis 5 000 %.

Bei Benutzung von Reaktoren mit Rühreinrichtung (Druckkessel, Autoklaven) und bei Anwendung eines Rohrreaktors zur kontinuierlichen Durchführung wird der Katalysator in Schlammform oder in fein pulverisierter Form benutzt und beispielsweise durch Schlammpumpen umgewälzt. Bei Anwendung der Rieselphase, aber auch bei Anwendung einer Pfropfströmung in einem Rohrreaktor kann der Katalysator auch als Festbettkatalysator, beispielsweise in tablettierter und geschichteter Form eingesetzt werden. Die Menge des Katalysators beträgt, gerechnet als Katalysatormetall, 0,01 bis 10 Gew.-%, bevorzugt 0,05 bis 8 Gew.-%, besonders bevorzugt 0,1 bis 5 Gew.-%, bezogen auf die Masse an umzusetzendem MSA. Im Falle einer Konti- oder Semibatch-Fahrweise beträgt die LHSV 10 bis 500 g, bevorzugt 20 bis 200 g, besonders bevorzugt 30 bis 100 g MSA pro g Katalysator pro Stunde.

Es ist zwar grundsätzlich möglich, die Verfahrenstemperatur innerhalb des genannten Bereiches während der Verfahensdurchführung zu variieren. Aus Gründen der Vereinfachung ist es jedoch bevorzugt, die Temperatur innerhalb eines sehr engen Bereiches im Gesamtbereich konstant zu halten. Damit entfällt jede spezielle, vom Reaktionsfortschritt abhängige Temperaturregelung. Ferner wird der Katalysator keinen Temperaturschwankungen ausgesetzt, wodurch seine Standzeit und Aktivität verlängert werden.

### Beispiel 1

In einem 0,7 l V4A-Autoklaven wurden 200 g BSA und 12 g Ra-Ni (dreimal mit Methanol, dreimal mit Toluol und anschließend mit BSA flüssig bei 130°C gewaschen) bei Raumtemperatur vorgelegt und unter 100 bar Wasserstoff bei 130°C aufgeschmolzen. Unter Rühren wurden anschließend innerhalb von 60 Minuten 200 g geschmolzenes MSA zugepumpt. Anschließend rührte man 20 Minuten nach, entspannte den Autoklaven und filtrierte heiß vom Katalysator ab. Nach dem Abkühlen erhielt man 401 g Feststoff mit einem Gehalt an BSA von 99,4 Gew.-%. Die Ausbeute, bezogen auf das eingesetzte MSA, betrug 97,3 % der Theorie.

### Beispiel 2

In einem 0,7 l V4A-Autoklaven wurden 200 g BSA und 6 g Pd-Katalysator auf Aluminiumoxid (5 Gew.-% Pd) bei Raumtemperatur vorgelegt und unter 100 bar Wasserstoff bei 130°C aufgeschmolzen. Unter Rühren wurden anschließend innerhalb von 60 Minuten 200 g geschmolzenes MSA zugepumpt. Anschließend rührte man 20 Minuten nach, entspannte den Autoklaven und filtrierte heiß vom Katalysator ab. Nach dem Abkühlen erhielt man 403 g Feststoff mit einem Gehalt an BSA von 99,2 Gew.-%. Die Ausbeute, bezogen auf das eingesetzte MSA, betrug 97,9 % der Theorie.

### Beispiel 3

In einem 0,7 l V4A-Autoklaven wurden 200 g BSA und 6 g Pd-Katalysator auf A-Kohle (5 Gew.-% Pd) bei Raumtemperatur vorgelegt und unter 100 bar Wasserstoff bei 130°C aufgeschmolzen. Unter Rühren wurden anschließend innerhalb von 60 Minuten 200 g geschmolzenes MSA zugepumpt. Anschließend rührte man 20 Minuten nach, entspannte den Autoklaven und filtrierte heiß vom Katalysator ab. Nach dem Abkühlen erhielt man 402 g Feststoff mit einem Gehalt an BSA von 99,8 Gew.-%. Die Ausbeute, bezogen auf das eingesetzte MSA, betrug 98,6 % der Theorie.

### Beispiel 4

In einem 0,7 l V4A-Autoklaven wurden 200 g BSA und 12 g Ra-Ni (dreimal mit Methanol, dreimal mit Toluol und anschließend mit BSA fl. bei 130°C gewaschen) bei Raumtemperatur vorgelegt und unter 100 bar Wasserstoff bei 130°C aufgeschmolzen. Unter Rühren wurden anschließend innerhalb von 60 Minuten 200 g geschmolzenes MSA zugepumpt. Anschließend rührte man 20 Minuten nach, stellte den Rührer ab und drückte über ein Steigrohr mit Fritte mittels des Wasserstoffdrucks ca. 200 g BSA ab. Anschließend wurden erneut 200 g MSA unter den oben genannten Bedingungen eingepumpt. Es werden insgesamt 1800 g MSA eingesetzt. Man isolierte 2020 g BSA mit einem Gehalt an BSA von 99,4 Gew.-%. Die Ausbeute betrug 98,4 % der Theorie, bezogen auf das eingesetzte MSA.

### Beispiel 5

In einem 0,7 l V4A-Autoklaven wurden 200 g BSA und 6 g Pd-Katalysator auf Al₂O₃ (5 Gew.-% Pd) bei Raumtemperatur vorgelegt und unter 100 bar Wasserstoff bei 130°C aufgeschmolzen. Unter Rühren wurden anschließend innerhalb von 60 Minuten 200 g geschmolzenes MSA zugepumpt. Anschließend rührte man 20 Minuten nach, stellte den Rührer ab und drückte über ein Steigrohr mit Fritte mittels des Wasserstoffdrucks ca. 200 g BSA ab. Anschließend wurden erneut 200 g MSA unter den oben genannten Bedingungen eingepumpt. Es werden insgesamt 2000 g MSA zugepumpt. Man isolierte 2240 g BSA mit einem Gehalt von 99,5 Gew.-%. Die Ausbeute, bezogen auf das eingesetzte MSA, betrug 99,4 % der Theorie, bezogen auf das eingesetzte MSA.

## Patentansprüche

1. Verfahren zur Herstellung von Bernsteinsäureanhydrid (BSA) durch katalytische Hydrierung von Maleinsäureanhydrid (MSA) mit Wasserstoff in flüssiger Phase, dadurch gekennzeichnet, daß ein Einsatzmaterial der Zusammensetzung 5 bis 100 Gew-.% MSA und 95 bis 0 Gew.-% BSA, bezogen auf das Gesamtgewicht von MSA und BSA, bei 120 bis 150°C und einem H₂-Druck von 10 bis 150 bar an einem Hydrier-Katalysator umgesetzt wird.

2. Verfahren nach Anspruch 1, dadurch gekennzeichnet, daß bei 125 bis 140°C umgesetzt wird.

3. Verfahren nach Anspruch 1, dadurch gekennzeichnet, daß bei 20 bis 130 bar umgesetzt wird.

4. Verfahren nach Anspruch 1, dadurch gekennzeichnet, daß in Gegenwart von 0,01 bis 10 Gew.-% bezogen auf die Masse an umzusetzendem MSA, an Katalysator, gerechnet als Katalysatormetall, umgesetzt wird.

5. Verfahren nach Anspruch 1, dadurch gekennzeichnet, daß in einer Semibatch-Fahrweise zu vorgelegtem flüssigen BSA flüssiges MSA zugesetzt und hydriert wird, daß BSA in der der zugesetzten MSA-Menge im Durchschnitt entsprechenden Menge dem Hydriergemisch entnommen wird, so daß noch BSA zurückbleibt, und der Vorgang unter erneutem Zusatz von MSA wiederholt wird.

6. Verfahren nach Anspruch 1, dadurch gekennzeichnet, daß das Einsatzmaterial kontinuierlich in der Rieselphase oder in einem Rohrreaktor umgesetzt wird.

7. Verfahren nach Anspruch 1, dadurch gekennzeichnet, daß der Hydrierkatalysator ein Raney-Skelettkatalysator ist, der 1 bis 4 der Elemente Ni, Co, Fe und Cu enthält.

8. Verfahren nach Anspruch 1, dadurch gekennzeichnet, daß der Hydrierkatalysator eine oder mehrere hydrieraktive Komponenten aus der I. und VIII. Nebengruppe enthält und als Metallpulver oder als Trägerkatalysator vorliegt.

9. Verfahren nach Anspruch 8, dadurch gekennzeichnet, daß als Träger Al₂O₃ der verschiedenen Strukturen, SiO₂ der verschiedenen Strukturen, Gemische aus Al₂O₃ und SiO₂, Aktivkohle, TiO₂ oder BaSO₄ in Frage kommen.

## Claims

1. Process for preparing succinic anhydride (SA) by catalytic hydrogenation of maleic anhydride (MA) with hydrogen in the liquid phase, characterized in that a starting material composed of from 5 to 100% by weight of MA and from 95 to 0% by weight of SA, based on the total weight of MA and SA, is reacted at from 120 to 150°C and an H₂ pressure of from 10 to 150 bar over a hydrogenation catalyst.

2. Process according to Claim 1, characterized in that the reaction is carried out at from 125 to 140°C.

3. Process according to Claim 1, characterized in that the reaction is carried out at from 20 to 130 bar.

4. Process according to Claim 1, characterized in that the reaction is carried out in the presence of from 0.01 to 10% by weight, based on the mass of MA to be reacted, of catalyst, calculated as catalyst metal.

5. Process according to Claim 1, characterized in that, in a semi-batch procedure, liquid MA is added to initially charged liquid SA and is hydrogenated, SA is taken from the hydrogenation mixture in an amount corresponding on average to the amount of MA added, so that some SA remains, and the procedure is repeated with renewed addition of MA.

6. Process according to Claim 1, characterized in that the starting material is reacted continuously in the trickling phase or in a tube reactor.

7. Process according to Claim 1, characterized in that the hydrogenation catalyst is a Raney skeleton catalyst containing from 1 to 4 of the elements Ni, Co, Fe and Cu.

8. Process according to Claim 1, characterized in that the hydrogenation catalyst contains one or more hydrogenation-active components from transition groups I and VIII and is present as metal powder or as supported catalyst.

9. Process according to Claim 8, characterized in that supports may be Al₂O₃ of the various structures, SiO₂ of the various structures, mixtures of Al₂O₃ and SiO₂, activated carbon, TiO₂ or BaSO₄.

## Revendications

1. Procédé de préparation d'anhydride succinique (AS) par hydrogénation catalytique d'anhydride maléique (AM) avec de l'hydrogène en phase liquide, qui est caractérisé en ce qu'on fait réagir un matériau de départ ayant pour composition, 5 à 100% en poids d'AM et 95 à 0% en poids d'AS, sur base du poids total de l'AM et de l'AS, entre 120 et 150°C et à une pression en H₂ allant de 10 à 150 bar et sur un catalyseur d'hydrogénation.

2. Procédé suivant la revendication 1, caractérisé en ce que l'on réalise la réaction à 125-140°C.

3. Procédé suivant la revendication 1, caractérisé en ce que l'on réalise la réaction à 20-130 bar.

4. Procédé suivant la revendication 1, caractérisé en ce que l'on réalise la réaction en présence de 0,01 à 10% en poids, sur base de la masse d'AM à transformer, d'un catalyseur, calculé comme catalyseur métallique.

5. Procédé suivant la revendication 1, caractérisé en ce que dans un mode de procédé en semi-continu, on ajoute à l'AS liquide disposé préalablement, l'AM liquide et on réalise l'hydrogénation, qu'on prélève l'AS en une quantité du mélange d'hydrogénation correspondant en moyenne à la quantité d'AM ajoutée, de sorte qu'il reste encore de l'AS, et le processus est répété avec une nouvelle addition d'AM.

6. Procédé suivant la revendication 1, caractérisé en ce que le matériau de départ est mis à réagir en continu, en phase de ruissellement ou dans un réacteur tubulaire.

7. Procédé suivant la revendication 1, caractérisé en ce que le catalyseur d'hydrogénation est un catalyseur à squelette Raney, qui contient 1 à 4 éléments parmi Ni, Co, Fe et Cu.

8. Procédé suivant la revendication 1, caractérisé en ce que le catalyseur d'hydrogénation contient un ou plusieurs composants actifs pour l'hydrogénation, parmi les groupes secondaires I et VIII et est présent sous forme de poudre métallique ou comme catalyseur supporté.

9. Procédé suivant la revendication 8, caractérisé en ce qu'on considère comme support, Al₂O₃ de différentes structures, SiO₂ de différentes structures, des mélanges de Al₂O₃ et de SiO₂, du charbon actif, TiO₂ ou BaSO₄.
